# EUROPEAN PATENT SPECIFICATION

(11) **EP 2 853 622 B1**
(45) Date of publication and mention of the grant of the patent: **13.01.2016**
(21) Application number: 13380041.7
(22) Date of filing: 30.09.2013
(51) Int. Cl.: C30B 29/14, A61L 27/12, A61K 6/033

(54) **Method of obtention of faceted fluorapatite nanocrystals**
Verfahren zur Gewinnung facettierter Fluorapatit-Nanokristalle
Procédé d'obtention de nanocristaux de fluorapatite à facettes

(43) Date of publication of application: 01.04.2015
(73) Proprietor: Universidade de Vigo, 36310 Vigo (ES)
(72) Inventor: González Fernández, Pio Manuel, 36208 Vigo (ES); Serra Rodríguez, Julia, 36211 Vigo (ES); López Álvarez, Miriam, 36209 Vigo (ES); Rodríguez Valencia, Cosme, 15883 Cacheiras Teo (ES); Balboa Franco, Maria Estela, 36141 Vilaboa (ES); Chiussi, Stefano, 36208 Vigo (ES)

(56) References cited:
- EP-A1- 2 287 122
- WO-A1-03/000588
- WO-A2-2007/118689
- CN-A- 101 879 332
- ZAPANTA LEGEROS ET AL: "Apatites in biological systems", PROGRESS IN CRYSTAL GROWTH AND CHARACTERIZATION, PERGAMON, OXFORD, GB, vol. 4, no. 1-2, 1 January 1981 (1981-01-01), pages 1-45, XP025844597, ISSN: 0146-3535, DOI: 10.1016/0146-3535(81)90046-0 [retrieved on 1981-01-01]

## Description

### Field of the Invention

The invention discloses the obtention of fluorapatite (FA) nanocrystals from enameloid sections of shark teeth for application as bone filler material in orthopedical surgery, enamel regeneration and other dental and maxillofacial treatments in the medical field.

### Background art

Hydroxyapatite (HA) is an apatite mineral corresponding to a calcium phosphate of chemical formula Ca₁₀(PO₄)₆(OH)₂. It is essentially a calcium phosphate of average Ca:P ratio of 1.67:1, containing hydroxyl groups. The structure is crystallized in the monoclinic or hexagonal system. Apatite materials in the form of HA are very demanded in bone tissue engineering for repairing, replacing or regenerating defects at human bones or teeth for showing a similar composition to the mineral part of the human bone.

The HA constituting the main compound of the inorganic part of bones presents a specific crystalline structure comprising a different crystal morphology, size and orientation than synthetic apatites. These bone derived biological apatites have low crystallinity due to the need to solubilize the few crystals at continuous dissolution and crystallization cycles for the constant bone regeneration.

Teeth represent the most highly mineralized and hard tissue in mammals, including humans. Their extraordinary mechanical properties are also due to a hierarchical specific arrangement of calcium-deficient carbonated hydroxyapatite (bioapatite) crystals. The compact outer part of human teeth is formed by the enamel, consisting of micron-sized bars of apatite in a specific disposal with a content of organic matrix of about 1% wt. Fluorapatite (FA), of chemical formula Ca₅(PO₄)₃F, presents greater stiffness, higher elastic modulus and greater hardness than HA. The incorporation of fluoride promotes the crystallization of calcium phosphate, improving the thermal stability and reducing the dissolution of the mineral. The FA of the shark teeth presents the particularity that incorporates fluorine into the lattice in a higher and more efficient manner than any other biological source, including human or bovine teeth.

The infrared (IR) spectrum of shark enameloid is known in the art, especially regarding the hydroxide and carbonate bands. Both shark enameloid and geo-apatite are rich in fluoride, showing the geo-apatite the highest fluoride concentration. There are, however, indications that the hydroxide concentration is also higher in the geo-apatite than in shark enameloid, which is unlikely. This can be explained by the much higher carbonate content, and partly also by the higher water content of shark enameloid.

The synthetic obtention of fluorapatite as dense and well-bonded fluoridated hydroxyapatite coatings with several amounts of fluorine Ca₁₀(PO₄)₆(OH)_{2_}xFₓ (FHA), was carried out on commercially available pure titanium by aerosol deposition using FHA powders, in order to investigate the effect of a fluorine content on the properties of saline solution and the coatings and how this fluorine may affect cellular differentiation (Hahn BD et al. "Effect of fluorine addition on the biological performance of hydroxyapatite coatings on Ti by aerosol deposition", Journal of Biomaterials Applications, 2011, 27(5), 587-594). Fluoride ions were successfully incorporated into the HA lattice for both the FHA powders and the FHA coatings replacing the hydroxyl groups, according to the formula: Ca₁₀(PO₄)₆(OH)₂₋ₓFₓ (x = 0.5, 1.0 or 1.5), and then tested with osteoblasts. A positive stimulation of the osteoblasts was proved. It is important to notice that the coating was composed by a HA doped with fluorine, and not by fluorapatite. The authors do not report any suggestions on the obtention of the fluorapatite nanostructures.

It has recently been published the fabrication of nanocrystalline fluorapatite with Mg substitutions in order to improve the poor thermostability and poor mechanical properties of synthetic hydroxyapatite, replacing it by fluorapatite (Kheradmandfard M, Fathi MH. "Fabrication and characterization of nanocrystalline Mg-substituted fluorapatite by high energy ball milling", Ceramics International, 2013, 39, 1651-1658). The substitution with fluorides may favour the calcium phosphate crystallization, improving the thermal stability and reducing the dissolution of the mineral. However, the obtained crystalline zones build up in aggregates, as shown in Figure 5 and page 1655 -3.3- of the document:
*"The morphology of powders indicates that each of them is composed of agglomerates with irregular shape and dimension distribution".*

These aggregates, typical of chemical sinthesis, present a worse behaviour as biocompatible material when compared to natural crystal apatites.

Fluorapatite has also been obtained from codfish bones using CaCl₂ and NaF solutions (Piccirillo C et al. "Extraction and characterisation of apatite- and tricalcium phosphate-based materials from cod fish bones", Materials Science and Engineering, 2013, C33, 103-110). However, the treatment of the bones in solution prior to their annealing changed the composition of the material.

Fluoride ions can reduce the formation of dental caries in an environment contaminated by bacteria and can improve bone formation by stimulation of osteoblastic activity. These properties are of great interest in dental and maxillofacial tissue engineering.

Chen aimed to mimick the natural anticaries ability of the tooth by developing new effective anticaries materials using fluorapatite nanorods or nanowires (Chen H et al, "Synthesis of fluorapatite nanorods and nanowires by direct precipitation from solution", Crystal Growth & Design, 2006, 6(6), 1504-1508). This publication discloses the conditions needed to synthesize fluorapatite nanorods of different size, shape and composition to obtain a crystalline conglomerate able to be incorporated into dental materials. Again, all these synthetic nanostructures are obtained in the form of aggregates, from which it is not possible to obtain isolated and faceted nanocrystals as per the present invention.

Shark tooth enameloid is built up of two distinct series of apatite crystals of different sizes and shapes (Kallaste T, Nemliher J. "Apatite varieties in extant and fossil vertebrate mineralized tissues", Journal of Applied Crystallography, 2005, 38, 587-594). The larger crystals amount up to 15% of the total and size 500-1000 A° aprox., while the smaller ones are of 400-500 A°. However, these structures are either not isolated in single monocrystals but organized in macrostructures and stacks containing crystalline FA. The presence of faceted FA nanocrystals in shark's enameloid has resulted entirely surprising.

LeGeros discloses attempts to investigate the components of shark dentine by pyrolysis at 600 °C and analysis of the obtained material with X-ray diffraction and infrared absorption spectroscopy (LeGeros R Z, "Apatites in biological systems", Progress in Crystal Growth and Characterization, 1981, 4, 1-45).

The problem of the art is then to obtain isolated and faceted fluorapatite nanocrystals from either a natural source of synthetic synthesis. The solution proposed by the present invention is to isolate the nanocrystals from shark teeth.

### Description of the Invention

The invention discloses the finding of a new source of fluorapatite nanocrystals obtained from the enameloid of shark teeth. This source presents the advantages of its biological origin and the important incorporation of fluorine into the enameloid structure in the form of fluorapatite as a biomineral phase with partial substitution of phosphate and carbonate hydroxides for fluoride ions. The enormous amount of material due to the continuous release of the teeth by sharks to the seabed for their renewal makes this source plenty, inexpensive and fully assured throughout the year. It can therefore be easily implemented on an industrial scale.

The microstructure of this biological source is composed at the inside of the tooth by dentin, a bone phase with apatite nanocrystals inlayed in an organic matrix of round 29%wt collagen, at a porous structure with dentin tubes of micrometer size. Within the enamel have been reported in the art some HA stratified structures with a certain fluorine content, although not faceted FA nanocrystals. As per in mammalians, structural elements of the shark's teeth occur in a highly hierarchical way. The hard and highly mineralized enameloid is at the outer layer of the teeth.

Thus, the present invention is a method of obtention of faceted fluorapatite nanocrystals, the method comprising the isolation of shark teeth enameloid, pyrolysis of said enameloid at a temperature of between 1000-1200°C, mechanical milling of the obtained material and the final separation of the nanocrystals.

One benefit of FA is a higher stability when compared with pure hydroxyapatite, being less soluble than the latter and suitable for being used in certain applications requiring long term stability in a biological environment. The incorporation of fluorides into the apatite lattice promotes calcium phosphate crystallization, improves thermal stability in a biological environment and reduces the dissolution of the mineral. Fluorapatite presents the ability to form a solid solution with fluoridated HA with partial substitutions of OH- by F-This is of interest since the replacement of F- brings a reduction in the crystal unit cell volume giving the chemical stability of the apatite lattice, improved by stronger electrostatic bonds between adjacent ions and the fluoride. All these represent technological advantages as bone filler material over HA and else materials used by the art.

### Brief description of the Figures

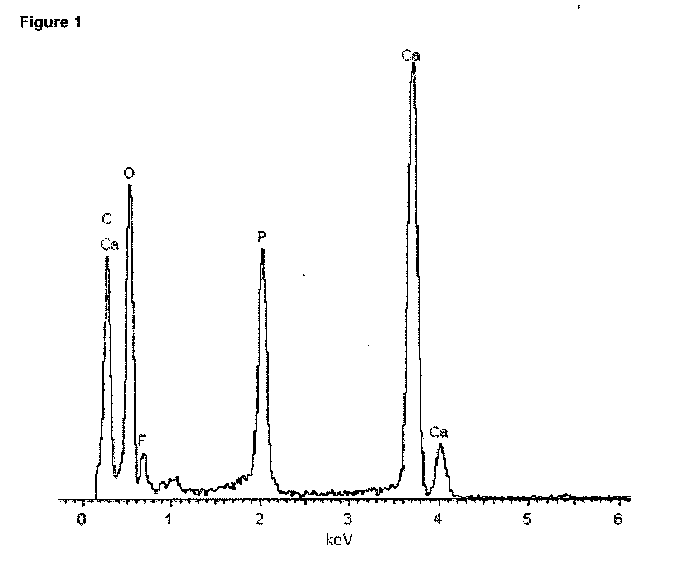

**Figure 1****:** X-ray spectroscopy (EDS) of fluoroapatite nanocrystals from enameloid shark teeth.

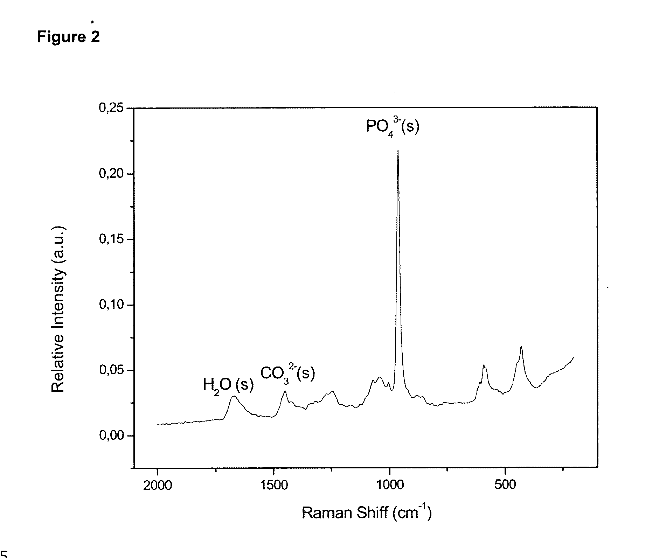

**Figure 2****:** FT-RAMAN of nanocrystals from enameloid shark teeth.

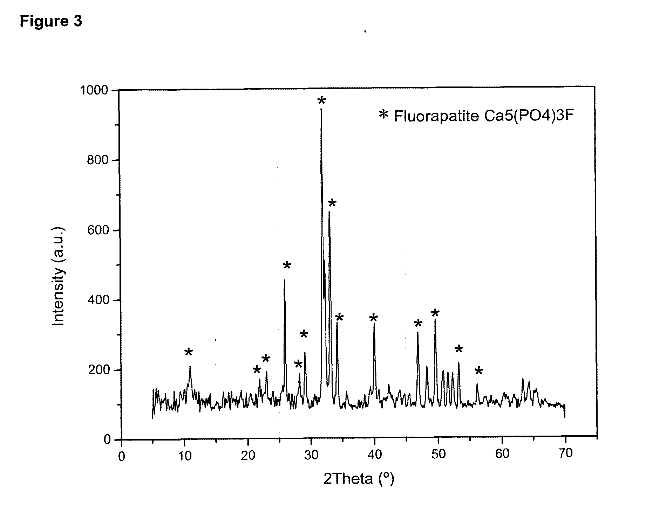

**Figure 3****:** X-Ray Diffraction (XRD) spectra of nanocrystals enameloid shark teeth.

### Detailed description of particular Embodiments

The following examples are provided in order to demonstrate and further illustrate certain preferred embodiments and aspects of the present invention, however not to be construed as limiting the scope thereof.

### EXAMPLES

### Example 1: Obtaining of nanocrystals from enameloid shark tooth.

One specimen of shark tooth from *Isurus oxyrinchus* specie was heated to 50°C during 15 minutes, and then immersed in liquid nitrogen for 5 seconds. The back and sharp sides of the tooth were worn-out with a fine drill and polishing tool until the dentin becomes visible. Then, a blow was inputted in the tooth sides slits resulting in that the enameloid was separated from the dentin. The obtained enameloid was subjected to pyrolysis at 1000°C during 12 hours, with a heating ramp of 2°C/min and an ending cooling ramp of 20°C/min; this process removed the remaining organic matter. The resulting white piece in the form of bulk was subjected to a ball mill (Retsch MM2000) during 5 minutes to obtain a powder of fluorapatite with crystals of 35 - 80 nm in one dimension per 50 - 700 nm in the other dimensions. Final obtention of the different sizes was achieved by centrifugation.

### Example 2: Crystal Characterization

The crystal composition of the nanocrystals obtained from the enameloid shark teeth was analyzed using Optical Emission Spectrometry with Inductively Coupled Plasma (ICP-OES; Perkin Elmer Optima 4300 DV) and ionic chromatography (Metrohm 733), being mainly composed by calcium, phosphate and fluoride. Magnesium and potassium were also detected but in trace concentrations. Results are given with respect to the total weight composition, the rest percentage up to the total corresponding to the organic matrix of the enameloid.

| **Crystals composition** | **Ca** | **P** | **F⁻** |
|---|---|---|---|
| (%) | 33.3 | 16.4 | 2.26 |

## Claims

1. A method of obtention of faceted fluorapatite nanocrystals, comprising the isolation of shark teeth enameloid, pyrolysis of said enameloid at a temperature of between 1000-1200°C, mechanical milling of the obtained material and final separation of said nanocrystals.

## Patentansprüche

1. Methode zur Erlangung facettierter Flourapatit Nanokristalle, einschließlich der Isolierung von Zahnschmelz aus Haifischzähnen, der Pyrolyse des besagten Zahnschmelz bei einer Temperatur von 1000-1200°C, dem mechanischen Mahlen des erhaltenen Materials sowie der Trennung der besagten Nanokristalle.

## Revendications

1. Une méthode d'obtention de nanocristaux de fluoroapatite facettés, qui comprend l'isolation d'émailoide de dent de requin, la pyrolyse de cet émailoide à une température entre 1000 et 2000°C, la moulure du matériau obtenu et la séparation finale de dits nanocrystaux.
